# EUROPÄISCHE PATENTSCHRIFT

(11) **EP 1 294 479 B1**
(45) Veröffentlichungstag und Bekanntmachung des Hinweises auf die Patenterteilung: **18.08.2004**
(21) Anmeldenummer: 01949432.7
(22) Anmeldetag: 23.06.2001
(51) Int. Cl.: B01J 13/16

(54) **VERFAHREN ZUR HERSTELLUNG AKTIVSTOFFHALTIGER KAPSELN MIT ULTRADÜNNER WANDSCHICHT**
METHOD FOR PRODUCING CAPSULES CONTAINING AN ACTIVE INGREDIENT AND HAVING AN ULTRA-THIN COATING
PROCEDE DE PRODUCTION DE CAPSULES CONTENANT UNE MATIERE ACTIVE ET A PAROI ULTRAMINCE

(30) Priorität: 30.06.2000 DE 10031132
(43) Veröffentlichungstag der Anmeldung: 26.03.2003
(73) Patentinhaber: Henkel Kommanditgesellschaft auf Aktien, 40589 Düsseldorf (DE)
(72) Erfinder: DREJA, Michael, 50931 Köln (DE); VON RYBINSKI, Wolfgang, 40593 Düsseldorf (DE); HOF, Matthias, 47249 Duisburg (DE); LEONHARD, Herbert, 46240 Bottrop (DE); REHAGE, Heinz, 45259 Essen (DE)
(86) Internationale Anmeldenummer: PCT/EP2001/007157
(87) Internationale Veröffentlichungsnummer: WO 2002/002222

(56) Entgegenhaltungen:
- EP-A- 0 322 820
- DE-A- 4 109 239
- GB-A- 2 055 739
- US-A- 4 428 983
- US-A- 4 439 581
- US-A- 4 640 709

## Beschreibung

Die vorliegende Erfindung betrifft ein Verfahren zur Herstellung aktiv- oder wirkstoffhaltiger, diffusionsdichter Polymerkapseln, -kügelchen oder - tröpfchen mit ultradünner Wandschicht durch Grenzflächenpolymerisation sowie die Verwendung der auf diese Weise hergestellten Kapseln, Kügelchen oder Tröpfchen als Delivery-Systeme für Aktiv- oder Wirkstoffe, insbesondere zum Einsatz in Kosmetikprodukten, pharmazeutischen Zusammensetzungen, Klebstoffen, Wasch- und Reinigungsmitteln und dergleichen.

Aktivstoffe oder Wirksubstanzen wie Duftstoffe, etherische Öle, Parfümöle und Pflegeöle, Farbstoffe oder pharmazeutisch aktive Wirkstoffe, die in kosmetischen und/oder pharmazeutischen Produkten oder in Wasch- und Reinigungsmitteln eingesetzt werden, verlieren häufig schon bei der Lagerung oder aber direkt bei der Anwendung ihre Aktivität. Manche dieser Stoffe können auch eine zur Verwendung nicht ausreichende Stabilität besitzen oder störende Wechselwirkungen mit anderen Produktbestandteilen verursachen.

Daher ist es von Interesse, solche Substanzen kontrolliert und am gewünschten Einsatzort mit maximaler Wirkung einzusetzen.

Aus diesem Grunde werden Aktiv- oder Wirksubstanzen wie Duftstoffe, Pflegeöle und antibakterielle Wirkstoffe den Produkten in räumlich abgegrenzter, geschützter Form zugesetzt. Häufig werden empfindliche Substanzen in Kapseln verschiedener Größen eingeschlossen, auf geeigneten Trägermaterialien adsorbiert oder chemisch modifiziert. Die Freisetzung kann dann mit Hilfe eines geeigneten Mechanismus aktiviert werden, beispielsweise mechanisch durch Scherung, oder diffusiv direkt aus dem Matrixmaterial erfolgen.

Daher werden Systeme gesucht, die sich als Verkapselungs-, Transport- oder Darreichungsvehikel - oft auch als "Delivery-Systeme" oder "Carrier-Systeme" bezeichnet - eignen.

Es existieren bereits zahlreiche kommerzielle Delivery-Systeme, die auf porösen Polymerpartikeln oder Liposomen basieren (z. B. Mikrosponges® von der Firma Advanced Polymer Systems oder aber Nanotopes® von der Firma Ciba-Geigy, siehe hierzu B. Herzog, K. Sommer, W. Baschong, J. Röding *"Nanotopes™: A Surfactant Resistant Carrier System"* in SÖFW-Journal, 124. Jahrgang 10/98, Seiten 614 bis 623).

Der Nachteil dieser herkömmlichen, aus dem Stand der Technik bekannten Delivery-Systeme besteht darin, daß sie nur ein geringes Beladungspotential aufweisen, die Partikelgröße der Polymerkugeln meist im Bereich von einigen Mikrometern bis einigen 100 µm liegt und eine Verkapselung der Wirksubstanzen in der Regel nicht in situ erfolgen kann. Die Modifizierung der Kapseloberflächen ist nicht möglich bzw. sehr aufwendig. Liposome besitzen außerdem eine für viele Anwendungen ungenügende Stabilität.

Es ist daher die Aufgabe der vorliegenden Erfindung, ein Verfahren zur Herstellung von polymeren Kapseln, Kügelchen oder Tröpfchen mit ultradünnen Wandschichten bereitzustellen, die sich als Träger für Aktiv- und Wirkstoffe verschiedenster Art eignen.

Insbesondere sollen durch ein solches Verfahren Kapseln, Kügelchen oder Tröpfchen hergestellt werden können, die als Delivery-Systeme für die genannten Aktiv- und Wirkstoffe verwendet werden können und somit eine kontrollierte Freisetzung dieser Aktiv- und Wirkstoffe am gewünschten Ort gewährleisten.

Die ultradünnen Wandschichten der hergestellten Kapseln, Kügelchen oder Tröpfchen sollen möglichst diffusionsdicht gegenüber dem eingeschlossenen Aktiv- oder Wirkstoff sein. Des weiteren soll durch die Verkapselung eine Koagulation, Agglomeration oder unkontrollierte Diffusion der eingeschlossenen Aktiv- oder Wirkstoffe verhindert und gleichzeitig deren kontrollierte Freisetzung ermöglicht werden.

Die nach dem erfindungsgemäßen Verfahren hergestellten Kapseln, Kügelchen oder Tröpfchen sollen weiterhin ein möglichst großes Beladungspotential aufweisen.

Weiterhin soll das Verfahren durch die Wahl der Herstellungsparameter, insbesondere der verwendeten Ausgangsstoffe (Edukte) und der Reaktionsbedingungen, eine gezielte Steuerung ("Maßschneidern") der Eigenschaften der erzeugten polymeren Kapseln, Kügelchen oder Tröpfchen ermöglichen.

Gelöst wird das der vorliegenden Erfindung zugrundeliegende Problem durch ein Verfahren zur Herstellung aktiv- oder wirkstoffhaltiger Polymerkapseln, - kügelchen oder -tröpfchen mit ultradünner Wandschicht, bei dem
(a) zunächst eine Dispersion hergestellt wird, die mindestens einen zu verkapselnden oder einzuschließenden Aktiv- oder Wirkstoff enthält und ausgehend von der durch radikalische Grenzflächenpolymerisation Polymerisate gebildet werden können;
(b) anschließend eine wärmeinduzierte, plasmainduzierte oder strahlungsinduzierte (z. B. durch Licht wie Laserlicht, durch Röntgenstrahlung, durch γ-Strahlung etc.) radikalische Grenzflächenpolymerisation in der in Verfahrensschritt (a) erhaltenen Dispersion durchgeführt wird, so daß auf diese Weise eine in-situ-Verkapselung oder ein in-situ-Einschluß des mindestens einen Aktiv- oder Wirkstoffs in den durch Grenzflächenpolymerisation erzeugten Polymerkapseln, -kügelchen oder -tröpfchen erfolgt; und
(c) schließlich die auf diese Weise erhaltenen aktiv- oder wirkstoffhaltigen Polymerkapseln, -kügelchen oder -tröpfchen gegebenenfalls abgetrennt werden können.
wobei die auf diese Weise hergestellten aktiv- oder wirkstoffhaltigen Polymerkapseln, - kügelchen oder -tröpfchen ein Verhältnis von Wandschichtdicke zu Kapseldurchmesser von 1 : 5000 bis 1 : 5, vorzugsweise von 1 : 1000 bis 1 : 10, insbesondere von 1 : 500 bis 1 : 100, sowie mittlere Teilchendurchmesser von 50 bis 50.000 nm, vorzugsweise von 100 bis 5.000 nm, ganz besonders bevorzugt von 100 bis 1.000 nm, aufweisen.

Gegenstand der vorliegenden Erfindung ist insbesondere ein Verfahren zur Herstellung aktiv- oder wirkstoffhaltiger Polymerkapseln, -kügelchen oder - tröpfchen mit ultradünner Wandschicht, das durch die folgenden Verfahrensschritte gekennzeichnet ist:
(a) Bereitstellung einer Dispersion, die
   - mindestens ein grenzflächenaktives (tensidisches, amphiphiles) Monomer,
   - mindestens einen zu verkapselnden oder einzuschließenden Aktivoder Wirkstoff,
   - gegebenenfalls mindestens einen Polymerisationsinitiator (Polymerisationsstarter),
   - gegebenenfalls mindestens ein Comonomer,
   - gegebenenfalls mindestens einen Polymerisationsbeschleuniger,
   - wäßrige Phase und
   - Ölphase (organische Phase)
   enthält;
(b) Durchführung einer wärme-, plasma- oder strahlungsinduzierten radikalischen Grenzflächenpolymerisation des mindestens einen tensidischen (amphiphilen, grenzflächenaktiven) Monomers in Gegenwart des mindestens einen Aktiv- oder Wirkstoffs und gegebenenfalls des mindestens einen Polymerisationsinitiators (Polymerisationsstarters), gegebenenfalls des mindestens einen Comonomers und gegebenenfalls des mindestens einen Polymerisationsbeschleunigers an der Phasengrenzfläche zwischen Ölphase (organischer Phase) und wäßriger Phase, wobei auf diese Weise eine in-situ-Verkapselung oder ein in-situ-Einschluß des mindestens einen Aktiv- oder Wirkstoffs bewirkt wird;
(c) gegebenenfalls Abtrennung der auf diese Weise erhaltenen aktiv- oder wirkstoffhaltigen Polymerkapseln, -kügelchen oder -tröpfchen aus dem Reaktionsgemisch.

Im Verfahrensschritt (a) wird zunächst eine Dispersion, vorzugsweise Emulsion, hergestellt, die mindestens ein grenzflächenaktives Monomer (in der vorliegenden Beschreibung synonym auch als "tensidisches Monomer" oder "amphiphiles Monomer" bezeichnet), mindestens einen Aktiv- oder Wirkstoff (in der vorliegenden Beschreibung synonym auch als "Aktiv- oder Wirksubstanz" bezeichnet), gegebenenfalls mindestens einen Polymerisationsinitiator (in der vorliegenden Beschreibung synonym auch als "Polymerisationsstarter" bezeichnet), gegebenenfalls mindestens ein Comonomer und gegebenenfalls mindestens einen Polymerisationsbeschleuniger enthält, wobei die Dispersion eine wäßrige Phase und ein Ölphase umfaßt.

Hierbei kann es sich entweder um eine Öl-in-Wasser-Dispersion bzw. Öl-in-Wasser-Emulsion oder aber um eine Wasser-in-Öl-Dispersion bzw. Wasser-in-Öl-Emulsion handeln.

Das Herstellen einer solchen Dispersion ist dem Fachmann geläufig. Üblicherweise wird bei der Herstellung der Dispersion so vorgegangen, daß zunächst der zu verkapselnde bzw. einzuschließende Aktiv- oder Wirkstoff in der Ölphase oder in der Wasserphase vorgelöst wird und anschließend die übrigen zuvor genannten Dispersionsbestandteile hinzugegeben werden. Gleichermaßen ist es möglich, zusammen mit dem Aktiv- oder Wirkstoff auch einen oder mehrere der übrigen Bestandteile (z. B. auch das grenzflächenaktive Monomer) in der Ölphase oder in der Wasserphase vorzulösen und dann die wäßrige Phase oder die Ölphase zuzugeben.

Nach Vereinigung aller Komponenten wird aus dem Ausgangsgemisch mit üblichen, dem Fachmann geläufigen Methoden oder Hilfsmitteln die Dispersion hergestellt. Hierzu können beispielsweise geeignete Dispergiergeräte verwendet werden, wie z. B. Ultraschallgeräte oder andere bekannte Dispergiergeräte zum Emulgieren, Suspendieren und Homogenisieren fließfähiger Medien (so z. B. ein Ultra-Turrax® von der Firma IKA-Maschinenbau). Die Vorgehensweise zur Herstellung der Dispersion ist nicht kritisch und ist dem Fachmann geläufig.

Gleichermaßen besteht die Möglichkeit, die Dispersion in einem kontinuierlichen Prozeß herzustellen. Dies ist besonders dann vorteilhaft, wenn der sich anschließende Verfahrensschritt (b) der Grenzflächenpolymerisation ebenfalls kontinuierlich betrieben wird. Eine solche Vorgehensweise ist dem Fachmann ebenfalls geläufig.

Die Dispergierzeit kann in weiten Grenzen variieren. Im allgemeinen beträgt sie 0,5 bis 3 min.

Durch die Art der Herstellung der Dispersion (Dispergierzeit, Art und Menge der zugeführten Energie wie Ultraschall etc.) kann - neben anderen variablen Parametern, wie im folgenden noch näher beschrieben - die mittlere Teilchengröße in der Dispersion und damit auch - die mittlere Teilchengröße des Endprodukts gesteuert werden.

Gegebenenfalls können der Dispersion noch weitere, übliche Zusatzstoffe oder Additive zugesetzt werden, deren Auswahl im Ermessen des Fachmanns liegt, so z. B. Tenside, Emulgatoren, Konsistenzgeber, Verdickungsmittel, Gelbildner, Stabilisatoren, Quellmittel etc.

Für die Dispersion verwendbare, erfindungsgemäß geeignete Ölphasen (organische Phasen) sind solche organischen Stoffe und Substanzen, die unter den Reaktionsbedingungen inert sind und den Reaktionsablauf nicht beeinträchtigen.

Beispiele für erfindungsgemäß geeignete Ölphasen sind höherwertige, lineare oder verzweigte, gesättigte oder ungesättigte, aliphatische, alicyclische oder aromatische Kohlenwasserstoffe, insbesondere solche mit mehr als 6 Kohlenstoffatomen, vorzugsweise solche mit mehr als 10 Kohlenstoffatomen, oder Mischungen solcher Kohlenwasserstoffe. Beispiele für solche Kohlenwasserstoffe sind aliphatische C₁₀-C₂₂-Kohlenwasserstoffe wie Dekan, Undekan, Dodekan, Tridekan, Tetradekan, Pentadekan, Hexadekan, Heptadekan, Octadekan, Nonadekan und Eikosan. Erfindungsgemäß ebenfalls geeignet sind die Kohlenwasserstoffe Tricyclodekan und Dekalin. Des weiteren können als erfindungsgemäß geeignete Kohlenwasserstoffe beispielsweise auch Squalan und Squalen eingesetzt werden.

Als Ölphase kommen erfindungsgemäß beispielsweise auch Guerbetalkohole auf Basis von Fettalkoholen mit 6 bis 18 Kohlenstoffatomen, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalinitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat in Betracht. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctylmalate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen, lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen und/oder Siliconöle.

Die Ölphase dient im allgemeinen als organische Trägerphase für die zu verkapselnden bzw. einzuschließenden Aktiv- oder Wirkstoffe. Gegebenenfalls kann auch die Wasserphase als Trägerphase für die Aktiv- oder Wirkstoffe dienen, und zwar im Falle von Aktiv- und Wirkstoffen, welche nicht in der organischen Trägerphase löslich sind.

Gemäß einer besonderen Ausführungsform, wenn der zu verkapselnde bzw. einzuschließende Aktiv- oder Wirkstoff selbst eine Ölphase darstellt, d. h. es sich bei dem zu verkapselnden bzw. einzuschließenden Aktiv- oder Wirkstoff um einen organischen Ölkörper handelt, kann die Dispersion auch ohne zusätzliche Ölphase hergestellt werden. Aber auch in einem solchen Fall ist es durchaus möglich, neben dem in Form eines Ölkörpers vorliegenden, zu verkapselnden bzw. einzuschließenden Aktiv- oder Wirkstoff eine zusätzliche Ölphase zur Herstellung der Dispersion zu verwenden, die als organische Trägerphase für den Aktiv- oder Wirkstoff dient.

Als wäßrige Phase dient herkömmliches (Leitungs-)Wasser oder aber auch deionisiertes Wasser. Bevorzugt ist deionisiertes Wasser. Zur Steuerung und Einstellung der Dichte der wäßrigen Phase können der wäßrigen Phase gezielt Zusatzstoffe oder Additive zugesetzt werden, so z. B. Salze.

Der Herstellung der Dispersion in Verfahrensschritt (a) schließt sich der Verfahrensschritt (b) an. Im Verfahrensschritt (b) wird das grenzflächenaktive Monomer in Gegenwart des zu verkapselnden oder einzuschließenden Aktivoder Wirkstoffs und gegebenenfalls in Gegenwart des Polymerisationsinitiators und gegebenenfalls in Gegenwart des Comonomers und gegebenenfalls in Gegenwart des Polymerisationsbeschleunigers einer radikalischen Grenzflächenpolymerisation an der Grenzfläche zwischen Ölphase und wäßriger Phase unterzogen. Die Grenzflächenpolymerisation erfolgt in der Regel wärme-, plasma- oder strahlungsinduziert, vorzugsweise lichtinduziert, insbesondere unter UV-Bestrahlung.

Wie zuvor erwähnt, kann die Grenzflächenpolymerisation gemäß Verfahrensschritt (b) entweder diskontinuierlich (z. B. als Batch-Verfahren) oder aber kontinuierlich durchgeführt werden.

Die Methode der Grenzflächenpolymerisation an sich ist bekannt und wird bereits seit langem angewandt. Es kann beispielsweise verwiesen werden auf den Übersichtsartikel von B. Tieke *"Polymerization at Interfaces"* in Polym. Organ. Media 1992, Seiten 105 bis 181, herausgegeben von C. M. Paleos Publisher: Gordon and Breach, Philadelphia. Des weiteren wird auf die in diesem Übersichtsartikel referierte Literatur verwiesen. Allgemein läßt sich die Grenzflächenpolymerisation als eine Polymerisation definieren, die in den Grenzflächen zweier miteinander nicht mischbarer Flüssigkeiten stattfindet.

Durch die radikalische Grenzflächenpolymerisation im Verfahrensschrift (b) des erfindungsgemäßen Verfahrens wird eine in-situ-Verkapselung bzw. ein in-situ-Einschluß des Aktiv- oder Wirkstoffs erzielt, so daß nach Beendigung der Polymerisationsreaktion aktiv- oder wirkstoffhaltige Polymerkapseln, - kügelchen oder -tröpfchen mit ultradünner Wandschicht entstehen, die den Aktiv- oder Wirkstoff in einer Polymermatrix einschließen und enthalten.

Wie zuvor erwähnt, verläuft die Polymerisation in Schritt (b) wärme-, plasmaund/oder strahlungsinduziert, insbesondere lichtinduziert. Wenn die Grenzflächenpolymerisation lichtinduziert erfolgt, können die Bestrahlungsdauern und die Bestrahlungsintensitäten in einem weiten Bereich variieren. Im allgemeinen arbeitet man in diesem Fall mit Bestrahlungsdauern von 5 bis 60 min. Die Bestrahlungsintensitäten betragen 5 bis 30 mW/m².

Es hat sich gezeigt, daß die lichtinduzierte radikalische Polymerisation besonders geeignet ist, um an der Grenzfläche zwischen Öl und Wasser entsprechende Monomere zu vernetzen. Zur Initiierung der Reaktion kann beispielsweise eine Quecksilber-Niederdruckdampflampe (z. B. vom Typ OS-RAM HNS 10 W/U ORF) verwendet werden. Solche Lampen zeichnen sich im allgemeinen durch eine hohe Photonausbeute, ein sehr genaues Emissionsspektrum bei einer Wellenlänge λ = 254 nm und eine sehr geringe Wärmeabstrahlung aus. Im allgemeinen arbeitet man bei Bestrahlungsmaxima von etwa 254 nm.

Wie bereits oben ausgeführt, ist auch die plasmainduzierte Grenzflächenpolymerisation erfindungsgemäß geeignet. Dazu kann z. B. ein Niedertemperaturplasma aus einem Mikrowellenreaktor oder aber ein Glühentladungsplasma verwendet werden. Für die Plasmainduktion kann z. B. ein HF-Generator mit 3,5 MHz verwendet werden. Bei dem Plasma kann es sich um ein Argon-Plasma handeln, welches beispielsweise bei 400 bis 1000 HPa gezündet wird, wobei die Temperatur in der Reaktionszone 10000 K beträgt. Geeignet ist beispielsweise eine N₂-Mischkammer mit Quenchröhre. Für weitere Einzelheiten zur Plasmainduktion kann verwiesen werden auf F. Sigenegar et al. in *Plasma Chem. Plasma Process.* 18 (1998), 153 sowie auf M. Guo und A. Ohl in *J. Phys. D. Appl. Phys.* 31 (1998), 2018.

Die Reaktionstemperatur für die Grenzflächenpolymerisation kann in weiten Grenzen variieren. Im allgemeinen liegt sie zwischen 10 °C und 100 °C, vorzugsweise zwischen 20 °C und 50 °C.

Die Monomerkonzentration in der Ausgangsmischung kann in weiten Grenzen variieren. Im allgemeinen beträgt die Monomerkonzentration in der Ausgangsmischung 5 bis 60 mmol/l, vorzugsweise 5 mmol/l bis 30 mmol/l, bezogen auf die Ausgangsmischung.

Erfindungsgemäß geeignete grenzflächenaktive (tensidische, amphiphile) Monomere sind insbesondere ausgewählt aus der Gruppe von grenzflächenaktiven (tensidischen, amphiphilen) (Meth-)Acrylaten, Succinaten und Sulfonaten sowie deren Derivaten.

Als Beispiele für erfindungsgemäß geeignete grenzflächenaktive Acrylate, Succinate und Sulfonate sowie deren Derivate lassen sich die folgenden Verbindungen nennen: Trimethylolpropantriacrylat (z. B. Photomer® 4006, vertrieben von der Firma Cognis GmbH Deutschland), Trimethylolpropanethoxylattriacrylat (z. B. Photomer® 4149, vertrieben von der Firma Cognis GmbH Deutschland), Trimethylolpropanpropoxylattriacrylat (z. B. Photomer® 4072, vertrieben von der Firma Cognis GmbH Deutschland), aliphatische Urethandiacrylate (z. B. Photomer® 6010, vertrieben von der Firma Cognis GmbH Deutschland), aliphatische Urethantriacrylate (z. B. Photomer® 6008, vertrieben von der Firma Cognis GmbH Deutschland), Dimerdioldimethacrylat, Dodekandiol-1,12-dimethacrylat, Laurylallylsulfosuccinat (z. B. TREM-LF-40®), Dodecyl-15 EO-acrylat (z. B. Blemmer ALE 800®, vertrieben von der Firma Nippon Oil & Fats Co., Ltd.), Dodecyl-15 EO-methacrylat (z. B. Blemmer PLE 800®, vertrieben von der Firma Nippon Oil & Fats Co., Ltd.), Octadecyl-15 EO-methacrylat (z. B. Blemmer PSE 800®, vertrieben von der Firma Nippon Oil & Fats Co., Ltd.), Octadecyl-15 EO-acrylat (z. B. Blemmer ASE 800®, vertrieben von der Firma Nippon Oil & Fats Co., Ltd.), Diallylammoniumdodecylsulfonat, Diallylsulfonium-2-hydroxydodecylchlorid, Diallylsulfonium-2-hydroxytetradecylchlorid, Diallylsulfonium-2-hydroxyhexadecylchlorid, Polyethylenglykolmonomethacrylat (z. B. Blemmer PE-Serie®, vertrieben von der Firma Nippon Oil & Fats Co., Ltd.), Polyethylenglykolmonoacrylat (z. B. Blemmer AE-Serie®, vertrieben von der Firma Nippon Oil & Fats Co., Ltd.), Polypropylenglykolmonomethacrylat (z. B. Blemmer PP-Serie®, vertrieben von der Firma Nippon Oil & Fats Co., Ltd.), Polypropylenglykolmonoacrylat (z. B. Blemmer AP-Serie®, vertrieben von der Firma Nippon Oil & Fats Co., Ltd.), Polyethylenglykolpolypropylenglykolmonomethacrylat (z. B. Blemmer PEP-Serie®, vertrieben von der Firma Nippon Oil & Fats Co., Ltd.), Polyethylenglykolpolypropylenglykolmonoacrylat (z. B. Blemmer AEP-Serie®, vertrieben von der Firma Nippon Oil & Fats Co., Ltd.) und deren Mischungen.

Weitere erfindungsgemäß geeignete grenzflächenaktive Monomere und ihre Synthese und Charakterisierung sind beispielsweise beschrieben bei P. Tundo, D. J. Kippenberger, N. J. Politi, P. Klahn und J. H. Fendler "*Redox Active Functionally Polymerized Surfactant Vesicles. Syntheses and Characterization*" in J. Am. Chem. Soc. **1982,** *104,* Seiten 5352 bis 5358.

Auch die Konzentration des oder der zu verkapselnden bzw. einzuschließenden Aktiv- oder Wirkstoffe in der Ausgangsmischung kann in weiten Bereichen variieren, und zwar in Abhängigkeit von der Aktivität der jeweiligen Aktiv- oder Wirkstoffe. Üblicherweise beträgt sie 0,001 bis 50 Gew-%, vorzugsweise 5 bis 40 Gew.-%, insbesondere 25 bis 40 Gew.-%, bezogen auf die Ausgangsmischung.

Der Aktiv- oder Wirkstoff liegt im allgemeinen .derart vor, daß er in der Ölphase als organischer Trägerphase gelöst ist; in diesem Fall wird der Aktivoder Wirkstoff zusammen mit der Ölphase eingeschlossen. Wenn der einzuschließende bzw. zu verkapselnde Aktiv- oder Wirkstoff selbst eine Ölphase ist, ist die Verwendung einer weiteren Ölphase nicht zwingend; in diesem Fall kann der Aktiv- oder Wirkstoff also entweder allein, d. h. in Masse oder Reinsubstanz, oder aber in einer in einer weiteren Ölphase gelösten Form verkapselt bzw. eingeschlossen werden.

Bei den erfindungsgemäß verwendeten Aktiv- oder Wirkstoffen kann es sich um Aktiv- oder Wirkstoffe beliebiger Art und Natur handeln. Jedoch sollten die verwendeten Aktiv- oder Wirkstoffe unter den Reaktionsbedingungen möglichst inert sein und den Reaktionsablauf nicht beeinträchtigen.

Nicht beschränkende Beispiele für erfindungsgemäß verwendbare Aktiv- oder Wirkstoffe sind die folgenden Substanzen und Substanzgemische: Duftstoffe; Öle wie etherische Öle, Parfümöle, Pflegeöle und Silikonöle; pharmazeutisch aktive Substanzen wie antibakterielle, antivirale oder fungizide Wirkstoffe; biogene Wirkstoffe; Antioxidantien; Vitamine und Vitaminkomplexe; Enzyme und enzymatische Systeme; kosmetisch aktive Substanzen wie beispielsweise Deodorantien (Desodorantien), Geruchsabsorber, Antitranspirantien, Haarpflege- und Haarfärbemittel, Antischuppenmittel, Hautpflegemittel oder andere für die Körperpflege einsetzbare Substanzen; UV-Lichtschutzfaktoren; Selbstbräuner; Konservierungsmittel; Insektenrepellentien; wasch- und reinigungsaktive Substanzen; biogene Wirkstoffe; Farbstoffe; Oxidations- und Bleichmittel; entschäumungsaktive Substanzen; Amine; sowie Mischungen der zuvor aufgeführten Aktiv- und Wirkstoffe.

Durch die Grenzflächenpolymerisation in Schritt (b) lassen sich an der Phasengrenze zwischen Öl (organischer Phase) und Wasser, insbesondere induziert durch Wärme oder geeignete UV-Bestrahlung, ultradünne Schichten erzeugen, wobei sich zu Beginn der Polymerisation kolloidale Aggregate bilden, die dann weiter vernetzen. Dieses Verhalten zeigte sich bei allen durchgeführten Reaktionen. Rheologische Untersuchungen haben gezeigt, daß man ein durchvernetztes System erhält, bei dem die elastischen Anteile gegenüber den viskosen Anteilen deutlich überwiegen. Außerdem konnten durch Amplitudentests hohe Grenzdeformationen bestimmt werden, welche ein fast kautschukähnliches Verhalten widerspiegeln.

Die Verwendung von radikalischen Polymerisationsinitiatoren (Polymerisationsstartem) bei der Durchführung von Verfahrensschritt (b) ist nicht zwingend, d. h. die Herstellung der erfindungsgemäßen Produkte kann mit oder ohne Verwendung eines Polymerisationsinitiators erfolgen. Jedoch lassen sich durch die Verwendung eines Polymerisationsinitiators die Reaktionszeiten zum Teil drastisch verkürzen, was insbesondere für die Vernetzung empfindlicher Aktiv- oder Wirkstoffe an der Phasengrenzfläche besonders vorteilhaft ist.

Wenn zur Durchführung des Grenzflächenpolymerisation ein Polymerisationsinitiator (Polymerisationsstarter) verwendet wird, kann die Konzentration des Polymerisationsinitiators (Polymerisationsstarters) in der Ausgangsmischung in weiten Grenzen variieren. Im allgemeinen beträgt sie 0,05 bis 0,5 mmol/l, vorzugsweise 0,05 bis 0,2 mmol/l, bezogen auf das Ausgangsgemisch.

Als radikalische Polymerisationsinitiatoren eignen sich alle dem Fachmann für diesen Zweck geläufigen Verbindungen. Dabei können sowohl Initiatoren verwendet werden, die sich in der organischen Phase lösen, als auch Initiatoren, die sich in der wäßrigen Phase lösen, aber auch Initiatoren, die sich an der Phasengrenzfläche befinden und somit grenzflächenaktiv sind.

Beispiele für in der organischen Phase lösliche Radikalstarter sind Azobisisobutyronitril (AIBN), 2,2-Dimethoxy-2-phenylacetophenon und Benzoinmethylether. Durch die Verwendung dieser Initiatoren lassen sich drastisch verkürzte Reaktionszeiten bei der Vernetzung an der Phasengrenzfläche erzielen. Die Verwendung dieser Initiatoren bietet somit die Möglichkeit, gezielt an der Grenzfläche eine Polymerisation zu starten.

Beispiele für in wäßriger Phase lösliche Radikalstarter sind Persulfatsalze (z. B. Natriumpersulfat) oder Übergangsmetallsulfate (z. B. Cer(IV)-sulfat). Initiatoren in wäßriger Phase, wie z. B. in wäßriger Phase gelöste Persulfatsalze, können an der Phasengrenze organische Monomere "einfangen", die durch UV-Bestrahlung erzeugt werden, und damit ebenfalls die Polymerisation beschleunigen.

Eine weitere Möglichkeit, gezielt an der Grenzfläche eine Polymerisation zu starten, ist die Verwendung eines Initiatorsystems, das amphiphile (tensidische, grenzflächenaktive) Substanzen enthält, was den Vorteil mit sich bringt, daß die grenzflächenaktiven Initiatoren direkt an der Grenzfläche die radikalische Polymerisationsreaktion starten, beschleunigen und stabilisieren. Solche grenzflächenaktive Starter werden auch als sogenannte "Inisurfs" bezeichnet. Auch diese Startersysteme bewirken eine deutliche Verkürzung der Reaktions- bzw. Bestrahlungszeit, d. h. also eine Reaktionsbeschleunigung bei gleichem Vernetzungsgrad. Beispiele für solche Inisurfs bzw. grenzflächenaktive, amphiphile Radikalstarter sind Reaktionsprodukte, die aus AIBN und nichtionischen Emulgatoren (wie z. B. Eumulgin® B1 [Polyoxyethylen -12-Cetylstearylalkohol], Eumulgin® B2 [Polyoxyethylen -20- Cetylstearylalkohol] und Eumulgin® B3 [Polyoxyethylen -30- Cetylstearylalkohol] von der Firma Henkel) synthetisiert werden.

Wie zuvor beschrieben, kann die in Schritt (a) hergestellte Dispersion gegebenenfalls mindestens ein geeignetes Comonomer enthalten, das unter den Reaktionsbedingungen in Schritt (b) des erfindungsgemäßen Verfahrens zusammen mit dem Monomer polymerisiert und das Kapselnetzwerk, d. h. die Wandschichten, bildet. Vorzugsweise handelt es sich bei dem gegebenenfalls verwendeten Comonomeren um ein grenzflächenaktives (amphiphiles, tensidisches) Comonomeres.

Wenn das erfindungsgemäße Verfahren unter Verwendung eines solchen Comonomers durchgeführt wird, erhält man Kapseln, Tröpfchen oder Kügelchen gleicher Art, die eine erhöhte Stabilität gegen Scherung aufweisen. Mit anderen Worten bewirkt die Verwendung eines Comonomers eine zusätzliche Stabilität der erzeugten Kapseln, Tröpfchen oder Kügelchen und sorgt für einen hohen Vernetzungsgrad.

Wenn das erfindungsgemäße Verfahren unter Verwendung eines Comonomers durchgeführt wird, beträgt die Comonomerkonzentration in der Ausgangsmischung 5 bis 40 mmol/l, vorzugsweise 5 bis 20 mmol/l.

Erfindungsgemäß geeignete Comonomere sind insbesondere ausgewählt aus Acrylsäuren und -derivaten (z. B. Tetraethylenglykoldiacrylaten, Tetrapropylenglykoldiacrylaten und deren Mischungen); Methacrylsäuren und -derivaten (z. B. Ethylenglykoldimethacrylaten, Triethylenglykoldimethacrylaten, Tetrapropylenglykoldimethacrylaten und deren Mischungen); Diallylaminen; sowie Diallylsulfiden.

Beispiele für erfindungsgemäß verwendbare Comonomere sind die folgenden Verbindungen: Methacrylsäure, Acrylsäure, Diallylamin, Diallylsulfid, Triethylenglykoldimethacrylat, Tetrapropylenglykoldimethacrylat (z. B. Blemmer PDP 200®, vertrieben von der Firma Nippon Oil & Fats Co., Ltd.), Ethylenglykoldimethacrylat (z. B. Blemmer PDE 50®, vertrieben von der Firma Nippon Oil & Fats Co., Ltd.), Tetrapropylenglykoldiacrylat (z. B. Blemmer ADP 200®, vertrieben von der Firma Nippon Oil & Fats Co., Ltd.) und Tetraethylenglykoldiacrylat (z. B. Blemmer ADE 200®, vertrieben von der Firma Nippon Oil & Fats Co., Ltd.).

Weitere, erfindungsgemäß geeignete Comonomer sind beschrieben in dem Artikel von B. Boutevin et al. *"Comparative de la réaction de polymérisation en solution de monomères tensioactifs méthacryliques"* in Eur. Polym. J., Vol. 32, No. 7, Seiten 821 bis 825 (1996).

Dem Verfahrensschritt (b) kann sich gegebenenfalls im Verfahrensschritt (c) eine Abtrennung bzw. Isolierung der im Verfahrensschritt (b) erhaltenen aktivoder wirkstoffhaltigen Polymerkapseln, -kügelchen oder -tröpfchen anschließen. Die Abtrennung kann nach dem Fachmann üblichen Methoden erfolgen, bei denen keine allzu großen Scherkräfte auf die Polymerkapseln, - kügelchen oder -tröpfchen ausgeübt werden, damit diese nicht beschädigt werden. Erfindungsgemäß geeignete Abtrennmethoden sind beispielsweise Gefriertrocknung (Lyophilisation) oder Sprühtrocknung unter schonenden Bedingungen.

Gleichermaßen ist es aber möglich, die in Verfahrensschritt (b) erhaltene Reaktionsmischung mit den erfindungsgemäßen aktiv- oder wirkstoffhaltigen Polymerkapseln, -kügelchen oder -tröpfchen direkt für die jeweilige Anwendung einzusetzen, gegebenenfalls nach Einengen bzw. Abziehen des oder der Dispersionsmittel(s).

Das erfindungsgemäße Verfahren eignet sich somit zur Verkapselung bzw. zum Einschluß von Aktiv- oder Wirksubstanzen in Polymerkapseln, - kügelchen oder -tröpfchen mit ultradünner diffusionsdichter Wandschicht. Der Begriff "diffusionsdicht" in diesem Zusammenhang meint, daß die ultradünnen Wandschichten der hergestellten Kapseln, Kügelchen oder Tröpfchen gegenüber dem eingeschlossenen Aktiv- oder Wirkstoff diffusionsdicht sind und eine Freisetzung nur kontrolliert und gezielt über einen geeigneten Freisetzungsmechanismus erfolgt (z. B. durch gezieltes Einwirken von Scherkräften).

Das erfindungsgemäße Verfahren ermöglicht somit eine effiziente Herstellung aktiv- oder wirkstoffhaltiger polymerer Kapseln, Kügelchen oder Tröpfchen mit ultradünner Wandschicht, deren Inhalt (Aktiv- oder Wirkstoff) gezieltfreigesetzt werden kann, insbesondere durch mechanische Zerstörung der polymeren Wandungen, z. B. durch Scherung.

Durch die Verkapselung bzw. den Einschluß wird ein Koagulation, Agglomeration und unkontrollierte Diffusion der eingeschlossenen Aktiv- oder Wirkstoffe verhindert und gleichzeitig deren kontrollierte Freisetzung (z. B. durch Scherung) ermöglicht.

Damit können die auch nach dem erfindungsgemäßen Verfahren hergestellten Polymerkapseln, -kügelchen oder -tröpfchen als Delivery-Systeme für die genannten Aktiv- oder Wirkstoffe verwendet werden und somit eine kontrollierte Freisetzung dieser Aktiv- oder Wirkstoffe am gewünschten Einsatzort gewährleisten. So eignen sich die nach dem erfindungsgemäßen Verfahren hergestellten Kapseln, Kügelchen oder Tröpfchen insbesondere als Delivery-Systeme im Bereich der Kosmetik, der Pharmazie, der Klebstoffverarbeitung und/oder der Wasch- und Reinigungsmittel.

Das erfindungsgemäße Verfahren eignet sich somit zur Herstellung von Polymerkapseln, -kügelchen oder -tröpfchen mit ultradünnen Wandschichten, die als Trägermatrix für Aktiv- oder Wirkstoffe verschiedenster Art geeignet sind. Nach dem erfindungsgemäßen Verfahren lassen sich aktiv- oder wirkstoffhaltige Polymerkapseln, -kügelchen oder -tröpfchen mit ultradünnen Wandschichten herstellen.

Der Begriff "ultradünn" in diesem Zusammenhang meint, daß sich im Idealfall monomolekulare Polymerschichten oder -filme bilden, die die zu verkapselnden bzw. einzuschließenden Aktiv- oder Wirkstoffe umgeben. In der Regel jedoch lassen sich aktiv- oder wirkstoffhaltige Polymerkapseln, -kügelchen oder -tröpfchen mit einem Wandschichtdicke/Kapseldurchmesser-Verhältnis von 1 : 5000 bis 1 : 5, vorzugsweise von 1 : 1000 bis 1 : 10, insbesondere von 1 : 500 bis 1 : 100, erhalten. Dabei weisen die auf diese Weise hergestellten aktiv- oder wirkstoffhaltigen Polymerkapseln, -kügelchen oder -tröpfchen im allgemeinen mittlere Teilchendurchmesser von 50 nm bis 50.000 nm, vorzugsweise von 100 nm bis 5.000 nm, ganz besonders bevorzugt von 100 nm bis 1.000 nm, auf.

Das erfindungsgemäße Verfahren eignet sich somit zur in-situ-Verkapselung oder zum in-situ-Einschluß von Aktiv- oder Wirkstoffen, die nach abgeschlossener Grenzflächenpolymerisation in Polymerkapseln, -kügelchen oder - tröpfchen mit ultradünner Wandschicht eingebettet sind.

Ein weiterer Vorteil der erfindungsgemäß herstellten Kapseln, Kügelchen oder Tröpfchen liegt darin, daß sie ein großes Beladungspotential gegenüber dem einzuschließenden bzw. zu verkapselnden Aktiv- oder Wirkstoffen aufweisen.

Ferner kann durch die Wahl der Herstellungs- und Reaktionsparameter (Art, Funktionalität und Menge der Monomere; Art, Funktionalität und Menge der Comonomere; Art und Menge der Aktiv- oder Wirkstoffe; Art und Menge der Polymerisationsinitiatoren; Reaktionsdauer und andere Reaktionsparameter, insbesondere Bestrahlungsdauer, -wellenlänge und -intensität; Art und Konzentration der Ölphase; Art und Konzentration der wäßrigen Phase; Variation der Teilchengröße in der Dispersion etc.) eine gezielte Steuerung, gewissermaßen ein "Maßschneidern" der Eigenschaften der erzeugten polymeren Kapseln, Kügelchen oder Tröpfchen ermöglicht werden. Durch die Wahl der Herstellungsparameter läßt sich die Formulierung der Mikrokapseln, - kügelchen oder -tröpfchen für die jeweilige Anwendung konfektionieren und optimieren.

Nach dem erfindungsgemäßen Verfahren lassen sich aktiv- oder wirkstoffhaltige Mikrokapseln, -kügelchen oder -tröpfchen mit deutlich verkürzten Reaktionszeiten darstellen. Daher stellt das erfindungsgemäße Verfahren eine neue und einfache Möglichkeit dar, selektiv wirksame Delivery-Systeme mit breitem Anwendungsprofil für zahlreiche Produkte zu erzeugen, insbesondere für den Bereich der Kosmetik und Körperpflege, der Pharmazie, der Klebstoffverarbeitung und/oder der Wasch- und Reinigungsmittel.

Die nach dem erfindungsgemäßen Verfahren hergestellten Produkte eignen sich insbesondere als Verkapselungs-, Transport- oder Darreichungsvehikel, d. h. als Delivery-Systeme oder Carrier-Systeme für verschiedenste Anwendungen (beispielsweise für den Bereich der Kosmetik und Körperpflege, für den Bereich der Pharmazie, für die Klebstoffverarbeitung und/oder zur Verwendung für die Wasch- und Reinigungsmittelindustrie).

Mit dem nach dem erfindungsgemäßen Verfahren hergestellten Delivery-Systeme gelingt die kontrollierte Freisetzung der eingeschlossenen bzw. verkapselten Aktiv- oder Wirkstoffe, so daß diese am gewünschten Einsatzort mit maximaler Wirkung eingesetzt werden können. Unter Umständen läßt sich hierbei eine gewisse Depotwirkung ausnutzen (z. B. für pharmazeutische Anwendungen). Auf diese Weise liefern die aktiv- oder wirkstoffhaltige Polymerkapseln, -kügelchen oder -tröpfchen eine effiziente Methode zur Kontrolle der Freisetzungskinetik, die durch die Wahl der verwendeten Herstellund Reaktionsparameter variiert werden kann, wie zuvor beschrieben. Gegenstand der vorliegenden Erfindung ist somit auch eine Methode oder ein Verfahren zur kontrollierten Freisetzung von Aktiv- oder Wirkstoffen.

Wie zuvor beschrieben, umfassen die Wandschichten der erfindungsgemäßen aktiv- oder wirkstoffhaltigen Polymerkapseln, -kügelchen oder -tröpfchen, die mindestens einen Aktiv- oder Wirkstoff eingeschlossen in einer Polymermatrix enthalten, ein Polymerisat, das durch radikalische Grenzflächenpolymerisation mindestens eines grenzflächenaktiven Monomers und gegebenenfalls mindestens eines Comonomers erhältlich ist. Das auf diese Weise gebildete polymere Kapselnetzwerk ist mindestens 4 Wochen lang stabil.

Der Aktiv- oder Wirkstoffgehalt in den erfindungsgemäßen Polymerkapseln, - kügelchen oder -tröpfchen beträgt etwa 1 bis etwa 99 Gew.-%, insbesondere etwa 10 bis etwa 80 Gew.-%, vorzugsweise etwa 50 bis etwa 80 Gew.-%, bezogen auf das Gesamtgewicht der Polymerkapseln, -kügelchen oder - tröpfchen.

Wie zuvor beschrieben, kann dabei der Aktiv- oder Wirkstoff in der Ölphase als organischer Trägerphase gelöst vorliegen, d. h. gemeinsam mit der Ölphase verkapselt sein. Gemäß einer besonderen Ausführungsform der vorliegenden Erfindung - nämlich dann, wenn der einzuschließende bzw. zu verkapselnde Aktiv- oder Wirkstoff selbst eine Ölphase darstellt - kann der Aktiv- oder Wirkstoff allein, d. h. in Masse oder Reinsubstanz, verkapselt bzw. eingeschlossen werden. Aber auch für den Fall, daß der einzuschließende bzw. zu verkapselnde Aktiv- oder Wirkstoff selbst eine Ölphase ist, besteht die Möglichkeit, den Aktiv- oder Wirkstoff in einer weiteren Ölphase zu lösen und ihn in der gelösten Form gemeinsam mit der Ölphase zu verkapseln.

Die vorliegende Erfindung wird anhand der folgenden Ausführungsbeispiele veranschaulicht, die die Erfindung jedoch keinesfalls beschränken.

### Ausführungsbeispiele

Die in der folgenden Tabelle aufgeführten Monomere wurden zur Erzeugung ultradünner Membranen an der Grenzfläche erfolgreich getestet.

| **Probe** | **Grenzflächenaktives Monomer** | **Handelsbezeichnung** |
|---|---|---|
| 1 | Trimethylolpropantriacrylat | Photomer® 4006 * |
| 2 | Trimethylolpropanethoxylattriacrylat | Photomer® 4149 * |
| 3 | Trimethylolpropanpropoxylattriacrylat | Photomer® 4072 * |
| 4 | Aliphatisches Urethandiacrylat | Photomer® 6010 * |
| 5 | Aliphatisches Urethantriacrylat | Photomer® 6008 * |
| 6 | Dimerdioldimethacrylat | |
| 7 | Dodekandiol-1,12-dimethacrylat | |
| 8 | Laurylallylsulfosuccinat | TREM-LF-40® ** |
| 9 | Dodecyl-15 EO-acrylat | Blemmer ALE 800® *** |
| 10 | Diallylammoniumdodecylsulfonat | |

| | | |
|---|---|---|
| * Die Produkte der Photomer®-Serie werden vertrieben von der Firma Cognis GmbH Deutschland. | | |
| ** TREM-LF-40® wird vertrieben von der Firma Cognis Corp., USA. | | |
| ***Blemmer ALE 800® wird vertrieben von der Firma Nippon Oil & Fats Co., Ltd. | | |

### Beispiel 1: Umsetzung ohne Initiatorsystem

Als Beispiel für die verschiedenen Umsetzungen ohne ein Initiatorsystem sind hier die Bedingungen der Versuche mit einigen Monomeren mit Dodekan als organischer Phase aufgeführt.

Das Mischungsverhältnis der organischen Phase bei dieser Versuchsreihe mit Wasser betrug jeweils 1 : 5. Alle Versuche wurden bei Raumtemperatur durchgeführt. Bei den Versuchen in anderen organischen Phasen wurde bei gleichen Konzentrationen gearbeitet.

Zuerst wurde aus Wasser und organischer Phase einschließlich des Monomers durch Behandlung mit einem Ultra-Turrax® eine stabile Emulsion erzeugt, die dann nach dem erfindungsgemäßen Verfahren polymerisiert wurde.

| **Versuch** | **Monomer** | **Ölphase** | **Monomerkonzentration** **(mmol/l)** | **UV- Intensität** **(mW/m**^{**2**}**)** | **Bemerkung** |
|---|---|---|---|---|---|
| 1 | Photomer® 4006 | Dodekan | 10 | 5 | Kapselnetzwerk nach 4 Wochen stabil |
| 2 | Photomer® 4072 | Dodekan | 15 | 8 | Kapselnetzwerk nach 4 Wochen stabil |
| 3 | Photomer® 6010 | Dodekan | 15 | 8 | Kapselnetzwerk nach 4 Wochen stabil |
| 4 | Photomer® 6008 | Dodekan | 10 | 8 | Kapselnetzwerk nach 4 Wochen stabil |

### Beispiel 2: Umsetzung mit Polymerisationsinitiator

Die Bedingungen für eine Reaktionsreihe mit 2,2-Dimethoxy-2-phenylacetophenon als Initiator in Dodekan als organischer Phase sind in der nachfolgenden Tabelle aufgeführt, wobei die Monomerkonzentration (10 mmol/l) und die UV-Intensität konstantgehalten wurden und die Bestrahlungsdauer variiert wurde. Der Initiator wurde unter diesen Versuchsbedingungen im Konzentrationsbereich von 0,2 bis 0,4 mmol/l verwendet.

| **Versuch** | **Monomer** | **Initiatorkonzentration** **(mmol/l)** | **Bestrahlungsdauer** **(min)** |
|---|---|---|---|
| 1 | Photomer® 4006 | 0,2 | 15 |
| 2 | Photomer® 4006 | 0,3 | 10 |
| 3 | Photomer® 4149 | 0,2 | 12 |
| 4 | Photomer® 4149 | 0,25 | 10 |
| 5 | Photomer® 4149 | 0,3 | 9 |
| 6 | Photomer® 4072 | 0,4 | 10 |

### Beispiel 3A:Herstellung eines grenzflächenaktiven Polymerisationsinitiators ausgehend von Eumulgin® B1 und Azobisisobutyronitril (AIBN)

0.2 mol Eumulgin®B1, 0,1 mol Azobisisobutyronitril (AIBN) und 650 g Toluol wurden in einer 1-1-Rührapparatur vermischt und in einem Kryostaten auf 2 °C abgekühlt. Dann wurde die Lösung unter Rühren mit HCl-Gas gesättigt. Anschließend ließ man über Nacht bei 0 °C rühren. Man goß auf ein Eis/Wasser-Gemisch, trennte die Toluolphase ab und trocknete über Natriumsulfat. Zum Schluß wurde das Lösungsmittel am Rotationsverdampfer bis zur Trockenheit abgezogen. Der fertige Starter wurde im Kühlschrank unter Stickstoff aufbewahrt.

### Beispiel 3B:Herstellung eines grenzflächenaktiven Polymerisationsinitiators ausgehend von Eumulgin® B2 und Azobisisobutyronitril (AIBN)

Man verfuhr wie unter Beispiel 3A, wobei man jedoch anstelle von Eumulgin® B 0,2 mol Eumulgin® B2 verwendete.

### Beispiel 4: Umsetzung mit einem grenzflächenaktiven Polymerisationsinitiator

Als Beispiel für die Herstellung von Kapseln durch Grenzflächenpolymerisation unter Verwendung grenzflächenaktiver Initiatoren ("Inisurfs") nach dem erfindungsgemäßen Verfahren wurden Umsetzungen des grenzflächenaktiven Monomers Blemmer ALE 800® mit einem Starter aus Eumulgin® B1 und AIBN und mit einem Starter aus Eumulgin® B2 und AIBN in Gegenwart von in Dodekan gelöstem Orangenöl durchgeführt.

Nach der Polymerisation durch UV-Bestrahlung erhielt man die Kapseln als eine quarkähnliche Masse, aus denen beim Verreiben die organische Wirkstoffphase (Orangenöl in Dodekan) wieder freigesetzt wurde.

In der nachfolgenden Tabelle sind die Daten aus den Umsetzungen mit den zwei zuvor genannten "Inisurfs" aufgeführt. Als Monomer wurde stets Blemmer ALE 800® verwendet.

| **Versuch** | **Inisurf** | **Initiatorkonzentration** **(mmol/l)** | **Bestrahlungsdauer** **(min)** |
|---|---|---|---|
| 1 | Eumulgin® B 1 + AIBN | 0,1 | 15 |
| 2 | Eumulgin® B 1 + AIBN | 0,15 | 13 |
| 3 | Eumulgin® B 1 + AIBN | 0,2 | 12 |
| 4 | Eumulgin® B2 + AIBN | 0, 1 | 10 |
| 5 | Eumulgin® B2 + AIBN | 0,15 | 9 |
| 6 | Eumulgin® B2 + AIBN | 0,2 | 7 |

### Beispiel 5: Umsetzung mit einem grenzflächenaktiven Polymerisationsinitiator

Die gleiche Versuchsreihe wie im vorangehenden Beispiel wurde unter Zusatz des Comonomers Triethylenglykoldimethacrylat erneut durchgeführt. Dabei erhielt man Kapseln gleicher Art, die eine erhöhte Stabilität gegen Scherung aufwiesen.

## Patentansprüche

1. Verfahren zur Herstellung aktiv- oder wirkstoffhaltiger Polymerkapseln, -kügelchen oder -tröpfchen mit ultradünner Wandschicht, bei dem
(a) zunächst eine Dispersion hergestellt wird, die mindestens einen zu verkapselnden oder einzuschließenden Aktiv- oder Wirkstoff enthält und ausgehend von der durch radikalische Grenzflächenpolymerisation Polymerisate gebildet werden können;
(b) anschließend eine wärme-, plasma- oder strahlungsinduzierte radikalische Grenzflächenpolymerisation in der in Verfahrensschritt (a) erhaltenen Dispersion durchgeführt wird, so daß auf diese Weise eine in-situ-Verkapselung oder ein in-situ-Einschluß des mindestens einen Aktiv- oder Wirkstoffs in den durch Grenzflächenpolymerisation erzeugten Polymerkapseln, -kügelchen oder - tröpfchen erfolgt; und
(c) schließlich die auf diese Weise erhaltenen aktiv- oder wirkstoffhaltigen Polymerkapseln, -kügelchen oder -tröpfchen gegebenenfalls abgetrennt werden können,
wobei die auf diese Weise hergestellten aktiv- oder wirkstoffhaltigen Polymerkapseln, - kügelchen oder -tröpfchen ein Verhältnis von Wandschichtdicke zu Kapseldurchmesser von 1 : 5000 bis 1 : 5, vorzugsweise von 1 : 1000 bis 1 : 10, insbesondere von 1 : 500 bis 1 : 100, sowie mittlere Teilchendurchmesser von 50 bis 50.000 nm, vorzugsweise von 100 bis 5.000 nm, ganz besonders bevorzugt von 100 bis 1.000 nm, aufweisen.

2. Verfahren nach Anspruch 1, **gekennzeichnet durch** die folgenden Verfahrensschritte:
(a) Bereitstellung einer Dispersion, enthaltend:
- mindestens ein grenzflächenaktives Monomer,
- mindestens einen zu verkapselnden oder einzuschließenden Aktiv- oder Wirkstoff,
- gegebenenfalls mindestens einen Polymerisationsinitiator,
- gegebenenfalls mindestens ein Comonomer,
- gegebenenfalls mindestens einen Polymerisationsbeschleuniger,
- wäßrige Phase und
- Ölphase;
(b) Durchführung einer wärme-, plasma- oder strahlungsinduzierten radikalischen Grenzflächenpolymerisation des mindestens einen grenzflächenaktiven Monomers in Gegenwart des mindestens einen Aktiv- oder Wirkstoffs und gegebenenfalls des mindestens einen Polymerisationsinitiators, gegebenenfalls des mindestens einen Comonomers und gegebenenfalls des mindestens einen Polymerisationsbeschleunigers an der Phasengrenzfläche zwischen Ölphase und wäßriger Phase, wobei **dadurch** eine in-situ-Verkapselung oder ein in-situ-Einschluß des mindestens einen Aktiv- oder Wirkstoffs erfolgt;
(c) gegebenenfalls Abtrennung der auf diese Weise erhaltenen aktiv- oder wirkstoffhaltigen Polymerkapseln, -kügelchen oder -tröpfchen aus dem Reaktionsgemisch.

3. Verfahren nach Anspruch 1 oder 2, wobei der Aktiv- oder Wirkstoff gleichzeitig die Ölphase darstellt.

4. Verfahren nach Anspruch 1 oder 2, wobei der Aktiv- oder Wirkstoff in der Ölphase und/oder in der wäßrigen Phase gelöst ist.

5. Verfahren nach einem der vorhergehenden Ansprüche, wobei die Grenzflächenpolymerisation lichtinduziert wird und die Bestrahlungsdauer vorzugsweise 5 bis 60 min bei einer Bestrahlungsintensität von 5 bis 30 mW/m² und bei einem Bestrahlungsmaximum von etwa 254 nm beträgt.

6. Verfahren nach einem der vorhergehenden Ansprüche, wobei die Reaktionstemperatur für die Grenzflächenpolymerisation 10 °C bis 100 °C, vorzugsweise 20 bis 50 °C, beträgt.

7. Verfahren nach einem der vorhergehenden Ansprüche, wobei die Monomerkonzentration in der Ausgangsmischung 5 bis 60 mmol/l, vorzugsweise 5 bis 30 mmol/l, beträgt.

8. Verfahren nach einem der vorhergehenden Ansprüche, wobei die Initiatorkonzentration in der Ausgangsmischung 0,05 bis 0,5 mmol/l, vorzugsweise 0,05 bis 0,2 mmol/l, beträgt.

9. Verfahren nach einem der vorhergehenden Ansprüche, wobei die Aktiv- oder Wirkstoffkonzentration in der Ausgangsmischung 0,001 bis 50 Gew.-%, vorzugsweise 5 bis 40 Gew.-%, insbesondere 25 bis 40 Gew.-%, beträgt, bezogen auf die Ausgangsmischung.

10. Verfahren nach einem der vorhergehenden Ansprüche, wobei die Dispergierzeit 0,5 bis 3 Minuten beträgt.

11. Verfahren nach einem der vorhergehenden Ansprüche, wobei der Aktiv- oder Wirkstoff ausgewählt wird aus der Gruppe von Duftstoffen; Ölen wie etherischen Ölen, Parfümölen, Pflegeölen und Silikonölen; pharmazeutisch aktiven Substanzen wie antibakteriellen, antiviralen oder fungiziden Wirkstoffen; Antioxidantien; Vitaminen und Vitaminkomplexen; Enzymen und enzymatischen Systeme; kosmetisch aktiven Substanzen; wasch- und reinigungsaktiven Substanzen; biogenen Wirkstoffen; Farbstoffen; Oxidations- und Bleichmitteln; entschäumungsaktiven Substanzen; Aminen; und deren Mischungen.

12. Verfahren nach einem der vorhergehenden Ansprüche, wobei das grenzflächenaktive Monomer ausgewählt wird aus der Gruppe von grenzflächenaktiven (Meth-)Acrylaten, Succinaten und Sulfonaten sowie deren Derivaten.

13. Verfahren nach Anspruch 12, wobei das grenzflächenaktive Monomer ausgewählt wird aus der Gruppe von Trimethylolpropantriacrylat, Trimethylolpropanethoxylattriacrylat, Trimethylolpropanpropoxylattriacrylat, aliphatischen Urethandiacrylaten, aliphatischen Urethantriacrylaten, Dimerdioldimethacrylat, Dodekandiol-1,12-dimethacrylat, Laurylallylsulfosuccinat, Dodecyl-15 EO-acrylat, Dodecyl-15 EO-methacrylat, Octadecyl-15 EO-methacrylat, Octadecyl-15 EO-acrylat, Diallylammoniumdodecylsulfonat, Diallylsulfonium-2-hydroxydodecylchlorid, Diallylsulfonium-2-hydroxytetradecylchlorid, Diallylsulfonium-2-hydroxyhexadecylchlorid, Polyethylenglykolmonomethacrylat, Polyethylenglykolmonoacrylat, Polypropylenglykolmonomethacrylat, Polypropylenglykolmonoacrylat, Polyethylenglykolpolypropylenglykolmonomethacrylat, Polyethylenglykolpolypropylenglykolmonoacrylat und deren Mischungen.

14. Verfahren nach einem der vorhergehenden Ansprüche, wobei der Polymerisationsinitiator ausgewählt wird aus der Gruppe von Azobisisobutyronitril (AIBN); Reaktionsprodukten aus AIBN und nichtionischen Emulgatoren wie Polyoxyethylen -12- Cetylstearylalkohol], Polyoxyethylen -20- Cetylstearylalkohol und Polyoxyethylen -30- Cetylstearylalkohol; Benzoinmethylether; 2,2-Dimethoxy-2-phenylacetophenon; Persulfaten wie Natriumpersulfat und Übergangsmetallsulfaten wie Cer(IV)-sulfat.

15. Verfahren nach einem der vorhergehenden Ansprüche, wobei das Comonomer ausgewählt wird aus Acrylsäuren und -derivaten, wie Tetraethylenglykoldiacrylaten, Tetrapropylenglykoldiacrylaten und deren Mischungen; Methacrylsäuren und - derivaten, wie Ethylenglykoldimethacrylaten, Triethylenglykoldimethacrylaten, Tetrapropylenglykoldimethacrylaten und deren Mischungen; Diallylaminen; und Diallylsulfiden.

16. Verfahren nach einem der vorhergehenden Ansprüche, wobei die auf diese Weise hergestellten aktiv- oder wirkstoffhaltigen Polymerkapseln, -kügelchen oder -tröpfchen einen Aktiv- oder Wirkstoffgehalt von 1 bis 99 Gew.-%, insbesondere von 10 bis 80 Gew.-%, vorzugsweise von 50 bis 80 Gew.-%, aufweisen, bezogen auf das Gesamtgewicht der Polymerkapseln, -kügelchen oder - tröpfchen.

17. Verfahren nach einem der vorhergehenden Ansprüche, wobei die Wandschicht der aktiv- oder wirkstoffhaltigen Polymerkapseln, -kügelchen oder -tröpfchen diffusionsdicht ist.

## Claims

1. Method for producing polymer capsules, pellets or droplets containing an active ingredient and having an ultra-thin coating, in which
(a) firstly a dispersion is prepared which comprises at least one active ingredient to be encapsulated or enclosed and, starting from which, polymers can be formed by free-radical interfacial polymerization;
(b) then a heat-, plasma- or radiation-induced free-radical interfacial polymerization is carried out in the dispersion obtained in step (a), such that, in this way, an in situ encapsulation or an in situ enclosure of the at least one active ingredient into the polymer capsules, pellets or droplets produced by interfacial polymerization takes place; and
(c) finally the polymer capsules, pellets or droplets containing an active ingredient obtained in this way can, if required, be separated off, where the polymer capsules, pellets or droplets which contain an active ingredient and have been prepared in this manner have a ratio of coating thickness to capsule diameter of from 1:5000 to 1:5, preferably from 1:1000 to 1:10, in particular from 1:500 to 1:100, and also have average particle diameters of from 50 to 50,000 nm, preferably from 100 to 5000 nm, very particularly preferably from 100 to 1000 nm.

2. Method according to Claim 1, **characterized by** the following steps:
(a) provision of a dispersion comprising:
- at least one interfacially active monomer,
- at least one active ingredient to be encapsulated or enclosed,
- optionally at least one polymerization initiator,
- optionally at least one comonomer,
- optionally at least one polymerization accelerator,
- aqueous phase and
- oil phase;
(b) carrying out a heat-, plasma- or radiation-induced free-radical interfacial polymerization of the at least one interface-active monomer in the presence of the at least one active ingredient and optionally the at least one polymerization initiator, optionally the at least one comonomer and optionally the at least one polymerization accelerator at the phase interface between oil phase and aqueous phase, which results in an in situ encapsulation or an in situ enclosure of the at least one active ingredient;
(c) if required, separation of the polymer capsules, pellets or droplets containing an active ingredient obtained in this way off from the reaction mixture.

3. Method according to Claim 1 or 2, where the active ingredient is also the oil phase.

4. Method according to Claim 1 or 2, where the active ingredient is dissolved in the oil phase and/or in the aqueous phase.

5. Method according to one of the preceding claims, where the interfacial polymerization is light-induced and the irradiation time is preferably 5 to 60 min at an irradiation intensity of from 5 to 30 mW/m² and at an irradiation maximum of about 254 nm.

6. Method according to one of the preceding claims, where the reaction temperature for the interfacial polymerization is 10°C to 100°C, preferably 20 to 50°C.

7. Method according to one of the preceding claims, where the monomer concentration in the starting mixture is 5 to 60 mmol/l, preferably 5 to 30 mmol/l.

8. Method according to one of the preceding claims, where the initiator concentration in the starting mixture is 0.05 to 0.5 mmol/l, preferably 0.05 to 0.2 mmol/l.

9. Method according to one of the preceding claims, where the active ingredient concentration in the starting mixture is 0.001 to 50% by weight, preferably 5 to 40% by weight, in particular 25 to 40% by weight, based on the starting mixture.

10. Method according to one of the preceding claims, where the dispersion time is 0.5 to 3 minutes.

11. Method according to one of the preceding claims, where the active ingredient is chosen from the group of fragrances; oils, such as essential oils, perfume oils, care oils and silicone oils; pharmaceutically active substances, such as antibacterial, antiviral or fungicidal active ingredients; antioxidants; vitamins and vitamin complexes; enzymes and enzymatic systems; cosmetically active substances; washing- and cleaning-active substances; biogenic active ingredients; dyes; oxidizing agents and bleaches; defoaming substances; amines; and mixtures thereof.

12. Method according to one of the preceding claims, where the interface-active monomer is chosen from the group of interface-active (meth)acrylates, succinates and sulphonates, and derivatives thereof.

13. Method according to Claim 12, where the interface-active monomer is chosen from the group of trimethylolpropane triacrylate, trimethylolpropane ethoxylate triacrylate, trimethylolpropane propoxylate triacrylate, aliphatic urethane diacrylates, aliphatic urethane triacrylates, dimerdiol dimethacrylate, dodecanediol-1,12 dimethacrylate, lauryl allyl sulphosuccinate, dodecyl-15 EO acrylate, dodecyl-15 EO methacrylate, octadecyl-15 EO methacrylate, octadecyl-15 EO acrylate, diallylammonium dodecylsulphonate, diallylsulphonium 2-hydroxydodecyl chloride, diallylsulphonium 2-hydroxytetradecyl chloride, diallylsulphonium 2-hydroxyhexadecyl chloride, polyethylene glycol monomethacrylate, polyethylene glycol monoacrylate, polypropylene glycol monomethacrylate, polypropylene glycol monoacrylate, polyethylene glycol polypropylene glycol monomethacrylate, polyethylene glycol polypropylene glycol monoacrylate and mixtures thereof.

14. Method according to one of the preceding claims, where the polymerization initiator is chosen from the group of azobisisobutyronitrile (AIBN); reaction products of AIBN and nonionic emulsifiers, such as polyoxyethylene-12 cetylstearyl alcohol, polyoxyethylene-20 cetylstearyl alcohol and polyoxyethylene-30 cetylstearyl alcohol; benzoin methyl ether; 2,2-dimethoxy-2-phenylacetophenone; persulphates, such as sodium persulphate and transition metal sulphates, such as cerium(IV) sulphate.

15. Method according to one of the preceding claims, where the comonomer is chosen from acrylic acids and derivatives, such as tetraethylene glycol diacrylates, tetrapropylene glycol diacrylates and mixtures thereof; methacrylic acids and derivatives, such as ethylene glycol dimethacrylates, triethylene glycol dimethacrylates, tetrapropylene glycol dimethacrylates and mixtures thereof; diallylamines; and diallyl sulphides.

16. Method according to one of the preceding claims, where the polymer capsules, pellets or droplets which contain an active ingredient and have been prepared in this way have an active ingredient content of from 1 to 99% by weight, in particular from 10 to 80% by weight, preferably from 50 to 80% by weight, based on the total weight of the polymer capsules, pellets or droplets.

17. Method according to one of the preceding claims, where the coating of the polymer capsules, pellets or droplets which contain an active ingredient is diffusion-tight.

## Revendications

1. Procédé de préparation de capsules, billes ou gouttes de polymère contenant une ou plusieurs substances actives, avec une couche de paroi ultra mince, dans lequel
(a) on prépare d'abord une dispersion, qui contient au moins une substance active à encapsuler ou à inclure et à partir de laquelle on peut préparer des polymères par polymérisation interfaciale radicalaire ;
(b) on effectue ensuite une polymérisation interfaciale radicalaire induite par voie thermique, par plasma ou par rayonnement de la dispersion obtenue dans l'étape de procédé (a), de telle manière qu'il se produit un encapsulage in situ ou une inclusion in situ de ladite au moins une substance active dans les capsules, billes ou gouttes de polymère obtenues par la polymérisation interfaciale ; et
(c) on peut enfin séparer, le cas échéant, les capsules, billes ou gouttes de polymère obtenues de cette manière, contenant une ou plusieurs substances actives,
les capsules, billes ou gouttes de polymère préparées de cette manière, contenant une ou plusieurs substances actives présentant un rapport épaisseur de couche de paroi à diamètre de capsule de 1:5000 à 1:5, de préférence de 1:1000 à 1:10, en particulier de 1:500 à 1:100, ainsi qu'un diamètre moyen des particules de 50 à 50000 nm, de préférence de 100 à 5000 nm, de manière tout à fait préférée de 100 à 1000 nm.

2. Procédé selon la revendication 1, **caractérisé par** les étapes de procédé suivantes :
(a) préparation d'une dispersion contenant :
- au moins un monomère tensioactif,
- au moins une substance active à encapsuler ou à inclure,
- le cas échéant au moins un initiateur de polymérisation,
- le cas échéant au moins un comonomère,
- le cas échéant au moins un accélérateur de polymérisation,
- une phase aqueuse et
- une phase huileuse ;
(b) réalisation d'une polymérisation interfaciale radicalaire induite par voie thermique, par plasma ou par rayonnement dudit au moins un monomère tensioactif en présence de ladite au moins une substance active et le cas échéant dudit au moins un initiateur de polymérisation, le cas échéant dudit au moins un comonomère et le cas échéant dudit au moins un accélérateur de polymérisation, à l'interface entre la phase huileuse et la phase aqueuse, suite à quoi il se produit un encapsulage in situ ou une inclusion in situ de ladite au moins une substance active ;
(c) le cas échéant séparation, du mélange réactionnel, des capsules, billes ou gouttes de polymère ainsi obtenues contenant une ou plusieurs substances actives.

3. Procédé selon la revendication 1 ou 2, dans lequel la substance active représente simultanément la phase huileuse.

4. Procédé selon la revendication 1 ou 2, dans lequel la substance active est dissoute dans la phase huileuse et/ou dans la phase aqueuse.

5. Procédé selon l'une quelconque des revendications précédentes, la polymérisation interfaciale étant induite par la lumière et la durée d'irradiation étant de préférence de 5 à 60 minutes à une intensité d'irradiation de 5 à 30 mW/m² et à un maximum d'irradiation d'environ 254 nm.

6. Procédé selon l'une quelconque des revendications précédentes, la température de réaction pour la polymérisation interfaciale étant de 10°C à 100°C, de préférence de 20°C à 50°C.

7. Procédé selon l'une quelconque des revendications précédentes, la concentration en monomère dans le mélange de départ étant de 5 à 60 mmoles/l, de préférence de 5 à 30 mmoles/l.

8. Procédé selon l'une quelconque des revendications précédentes, la concentration en initiateur dans le mélange de départ étant de 0,05 à 0,5 mmoles/l, de préférence de 0,05 à 0,2 mmoles/l.

9. Procédé selon l'une quelconque des revendications précédentes, la concentration en substance active dans le mélange de départ étant de 0,001 à 50% en poids, de préférence de 5 à 40% en poids, en particulier de 25 à 40% en poids, par rapport au mélange de départ.

10. Procédé selon l'une quelconque des revendications précédentes, le temps de dispersion étant de 0,5 à 3 minutes.

11. Procédé selon l'une quelconque des revendications précédentes, la substance active étant choisi dans le groupe des arômes ; des huiles telles que les huiles essentielles, les huiles parfumées, les huiles de soin et les huiles de silicone ; des substances pharmaceutiquement actives, telles que les additifs antibactériens, antiviraux ou fongicides ; des antioxydants ; des vitamines et des complexes vitaminés ; des enzymes et des systèmes enzymatiques ; des substances cosmétiquement actives; des substances actives de lavage et de nettoyage ; des additifs biogènes ; des colorants ; des oxydants et des agents de blanchiment ; des substances actives antimousses ; des amines ; et leurs mélanges.

12. Procédé selon l'une quelconque des revendications précédentes, le monomère tensioactif étant choisi dans le groupe des (méth)acrylates tensioactifs, des succinates tensioactifs et des sulfonates tensioactifs ainsi que leurs dérivés.

13. Procédé selon la revendication 12, le monomère tensioactif étant choisi dans le groupe du triacrylate de triméthylolpropane, du triacrylate de l'éthoxylate de triméthylolpropane, le triacrylate du propoxylate de triméthylolpropane, les diacrylates d'uréthanne aliphatiques, les triacrylates d'uréthanne aliphatiques, le diméthacrylate de diol dimère, le 1,12-diméthacrylate de dodécanediol, l'allylsulfosuccinate de lauryle, l'acrylate de 15-OE-dodécyle, le méthacrylate de 15 OE-dodécyle, le méthacrylate de 15 OE-octadécyle, l'acrylate de 15 OE-octadécyle, le dodécylsulfonate de diallylammonium, le 2-hydroxydodécylchlorure de diallylsulfonium, le 2-hydroxytétradécylchlorure de diallylsulfonium, le 2-hydroxyhexadécylchlorure de diallylsulfonium, le poly(monométhacrylate d'éthylèneglycol), le poly(monoacrylate d'éthylèneglycol), le poly(monométhacrylate de propylèneglycol), le poly(monoacrylate de propylèneglycol), le polyéthylèneglycol-poly(monométhacrylate de propylèneglycol), le polyéthylèneglycol-poly(monoacrylate de propylèneglycol) et leurs mélanges.

14. Procédé selon l'une quelconque des revendications précédentes, l'initiateur de polymérisation étant choisi dans le groupe de l'azobisisobutyronitrile (AIBN); des produits de réaction d'AIBN et d'émulsifiants non ioniques tels que le polyoxyéthylène-12-alcool cétylstéarylique, le polyoxyéthylène-20-alcool cétylstéarylique, le polyoxyéthylène-30-alcool cétylstéarylique ; le benzoïneméthyléther ; la 2,2-diméthoxy-2-phénylacétophénone ; les persulfates, tels que le persulfate de sodium et les sulfates des métaux de transition tels que le sulfate de cérium (IV).

15. Procédé selon l'une quelconque des revendications précédentes, le comonomère étant choisi parmi les acides acryliques et les dérivés acryliques, tels que les diacrylates de tétraéthylèneglycol, les diacrylates de tétrapropylèneglycol et leurs mélanges ; les acides méthacryliques et les dérivés méthacryliques, tels que les diméthacrylates d'éthylèneglycol, les diméthacrylates de triéthylèneglycol, les diméthacrylates de tétrapropylèneglycol et leurs mélanges ; les diallylamines et les sulfures de diallyle.

16. Procédé selon l'une quelconque des revendications précédentes, les capsules, billes ou gouttes préparées de cette manière, contenant une ou plusieurs substances actives présentant une teneur en substance active de 1 à 99% en poids, en particulier de 10 à 80% en poids, de préférence de 50 à 80% en poids, par rapport au poids total des capsules, billes ou gouttes de polymère.

17. Procédé selon l'une quelconque des revendications précédentes, la couche de paroi des capsules, billes ou gouttes de polymère contenant une ou plusieurs substances actives étant imperméable à la diffusion.
